# EUROPÄISCHE PATENTANMELDUNG

(11) **EP 0 652 201 A1**
(43) Veröffentlichungstag der Anmeldung: **10.05.1995**
(21) Anmeldenummer: 94116513.6
(22) Anmeldetag: 20.10.1994
(51) Int. Cl.: C07C 45/79, C07C 45/78, C07C 47/04

(54) **Verfahren zur Gewinnung konzentrierter wässriger Formaldehydlösungen durch Pervaporation**

(30) Priorität: 30.10.1993 DE 4337231
(71) Anmelder: BASF Aktiengesellschaft, D-67063 Ludwigshafen (DE)
(72) Erfinder: Dams, Albrecht, Dr., D-67157 Wachenheim (DE); Fried, Andreas, Dr., D-67240 Bobenheim-Roxheim (DE); Hammann, Fredy, D-67256 Weisenheim (DE)

(57) **Zusammenfassung**

Verfahren zur Gewinnung konzentrierter wäßriger Formaldehydlösungen durch Pervaporation, wobei ein wäßriges Formaldehydgemisch über eine hydrophile, porenfreie Membran geführt wird und selektiv Wasser über die Membran entfernt wird.

## Beschreibung

Die Erfindung betrifft ein Verfahren zur Gewinnung konzentrierter wäßriger Formaldehydlösungen durch Pervaporation.

Die destillative Aufkonzentrierung von wäßrigen Formaldehydlösungen wird durch das azeotrope Siedeverhalten erschwert (siehe Ind. Eng. Chem. Process Des. Dev. Jahrgang 1980, Seiten 179 - 185, "Isothermal Vapor-Liquid Equilibria for the Water-Formaldehyde System. A Predictive Thermodynamic Model 1"). Technisch eingesetzt wird die Zweidruckdestillation, bei der die Druckabhängigkeit der azeotropen Zusammensetzung ausgenutzt wird (siehe Ullmanns Encyklopädie der technischen Chemie, 4. Auflage, Band 11, Seiten 687 - 701). Nachteilig ist hierbei der hohe Energiebedarf und der apparative Aufwand des Destillationsverfahrens. Darüber hinaus ist die Formaldehydkonzentrierung aufgrund der Polymerisationsneigung in der wäßrigen Lösung auf 50 - 60 Gew.-% Formaldehyd begrenzt.

Bekannt ist die Entwässerung organischer Flüssigkeitsgemische mit azeotropem Siedeverhalten durch Pervaporation. Beispiele sind die Wasserabtrennung aus Alkoholen, Ketonen, Estern u.a., beschrieben beispielsweise in J. of Membrane Sci. 51, Jahrgang 1990, Seiten 169 - 179 "Pervaporation of liquid mixtures through polyvinylalcohol (PVA) membranes".

Bei der Pervaporation wird Wasser durch eine Membran, die hydrophile funktionale Gruppen enthält, aus dem Gemisch entfernt und im Permeat angereichert. Das treibende Gefälle wird durch Absenken des Wasserdampfpartialdrucks auf der Permeatseite beispielsweise durch Evakuieren, erzeugt. Die nicht über die Membran abgetrennten organischen Komponenten werden aufkonzentriert.

Durch Pervaporation können Gemische in einem Verfahrensschritt entwässert werden, ohne daß die Trennung durch Azeotrope, wie bei der Destillation, limitiert ist.

Der vorliegenden Erfindung liegt demzufolge die Aufgabe zugrunde, die Pervaporation in einem Verfahren zur Aufkonzentrierung von wäßrigen Formaldehydlösungen einzusetzen. Ziel ist die Erfindung eines Verfahrens, das im energetischen und apparativen Aufwand und in den erreichbaren Reinheiten einen Fortschritt gegenüber dem Zweidruckdestillationsverfahren darstellt.

Diese Aufgabe wird erfindungsgemäß dadurch gelöst, daß ein wäßriges Formaldehydgemisch über eine hydrophile, porenfreie Membran geführt wird und selektiv Wasser über die Membran entfernt wird.

Dabei gilt es, mehrere Probleme der Pervaporation bei der Formaldehydkonzentrierung zu lösen, die bislang als verfahrensentscheidende Hindernisse angesehen werden:
- Membranbeständigkeit gegen konzentrierte wäßrige Formaldehydlösungen
- Formaldehydrückhaltung auch bei höheren Wassergehalten und damit stärkerer Membranquellung
- Polymerisatbildung bei höheren Formaldehydkonzentrationen und dadurch bedingte Belegung der Membran und Verstopfung der Strömungswege.

Darüber hinaus weicht die Konzentrierung von wäßrigen Formaldehydlösungen von bisherigen Anwendungen der Pervaporation ab, bei denen das Verfahren lediglich zur Restentwässerung von Gemischen eingesetzt wird und der Anteil organischer Komponenten in der Rohlösung bereits hoch ist.

Die oben aufgezeigten Probleme werden beseitigt, durch
- Einsatz einer geeigneten Membran,
- Einstellung geeigneter Prozeßbedingungen und
- Wahl geeigneter Verfahrensverschaltungen,
wie in den nachfolgenden Ausführungsbeispielen der Erfindung näher beschrieben wird, die die wesentlichen erfinderischen Merkmale enthalten.

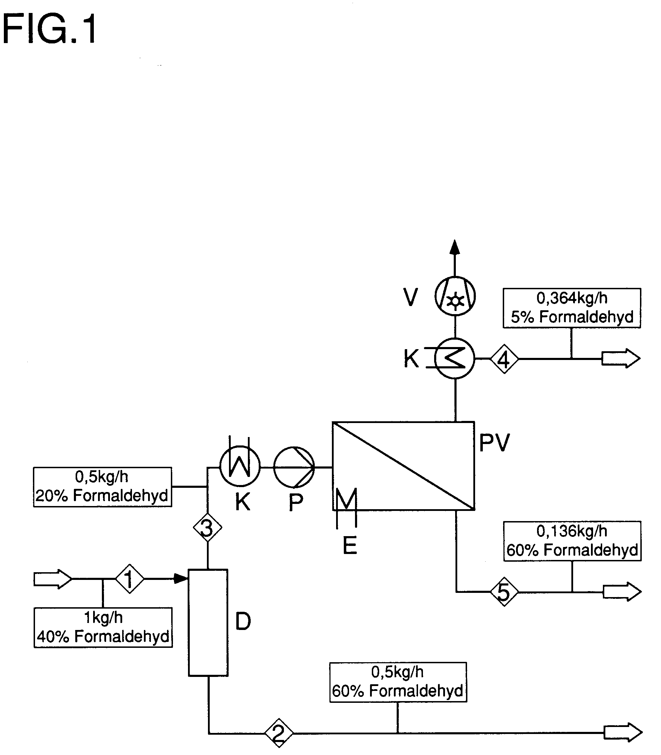

Figur 1 zeigt die Gewinnung konzentrierter wäßriger Formaldehydlösungen durch Verschaltung von Destillation und Pervaporation.

Die Formaldehyd-Rohlösung (Strom 1) wird in einer Destillationseinheit (D) aufgetrennt in eine konzentrierte Lösung (Strom 2) und ein verdünntes Destillat (Strom 3). Aus dem Destillat wird durch Abtrennung eines wasserreichen Stroms (Stroms 4) = Permeat mittels Pervaporation (PV) Formaldehyd als konzentrierte Lösung (Strom 5) zurückgewonnen. Die verfahrenstechnischen Vorteile sind
- eine Steigerung der Ausbeute durch die nachgeschaltete Pervaporation
- Möglichkeit zur Direktentsorgung des wasserreichen Permeatstroms (Strom 4) aufgrund des weiter reduzierten Formaldehydgehaltes
- die Möglichkeit zur Ergänzung bestehender Destillationseinheiten
Es bedeuten
- K =: Kondensatoren
- P =: Pumpe
- E =: Erhitzer und
- V =: Vakuumpumpe

### Beispiel 1

Mengen und Zusammensetzungen der einzelnen Ströme sind in Figur 1 angegeben. Die Destillation wird bei einem Druck von 600 mbar und einer Temperatur von 80°C betrieben, die Pervaporation ebenfalls bei 80°C. Auf der Permeatseite wird ein Druck von 20 mbar gehalten. Verwendet wird eine Membran mit einer thermisch vernetzten Trennschicht aus Polyvinylalkohol und einer Unterstruktur aus Polyacrylnitril, die eine hohe Wasserselektivität und eine gute Beständigkeit aufweist.

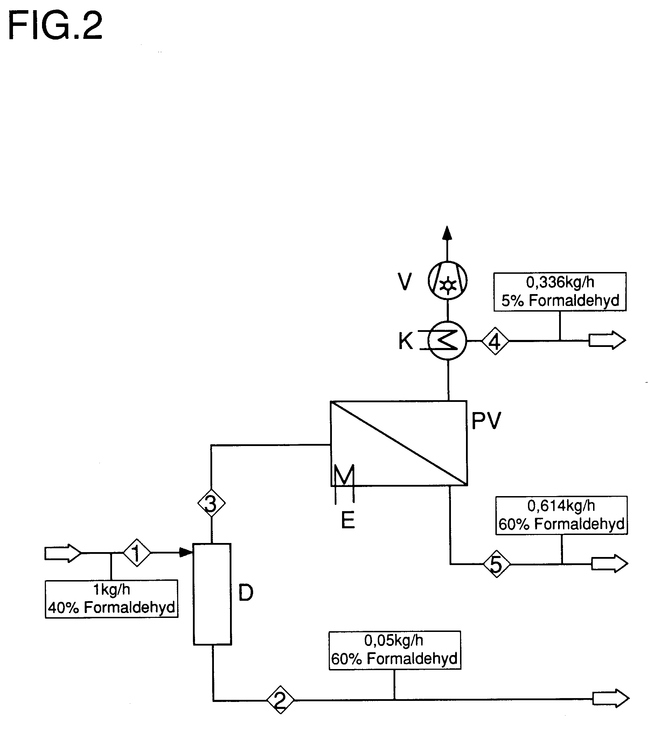

Figur 2 zeigt die Gewinnung konzentrierter wäßriger Formaldehydlösungen durch Verschaltung von Destillation und Pervaporation mit dampfförmigem Zulauf (Dampfpermeation).

Im Unterschied zu der Ausführung in Figur 1 wird das Destillat nicht auskondensiert, sondern dampfförmig über die Membran geführt. Die zusätzlichen Vorteile dieser Ausführungsvariante gegenüber der vorherigen sind, daß
- aufgrund einer geringeren Membranquellung bei dampfförmigem Zulauf höhere Permeatreinheiten erreichbar sind,
- eine Kondensation und erneute Verdampfung des Destillates vermieden wird,
- in der Dampfphase höhere Formaldehydkonzentrationen (> 60 Gew.-%) möglich sind, ohne Beeinträchtigung des Prozesses durch Polymerisation.

### Beispiel 2

Mengen und Zusammensetzungen der einzelnen Ströme sind in Figur 2 angegeben. Die Destillation wird bei einem Druck von 900 mbar und einer Temperatur von 90°C betrieben, die Dampfpermeation bei einer geringfügig höheren Temperatur zur Vermeidung von Kondensatbildung auf der Membran. Auf der Permeatseite wird ein Druck von 20 mbar gehalten. Verwendet wird das gleiche Membranmaterial wie im ersten Ausführungsbeispiel.

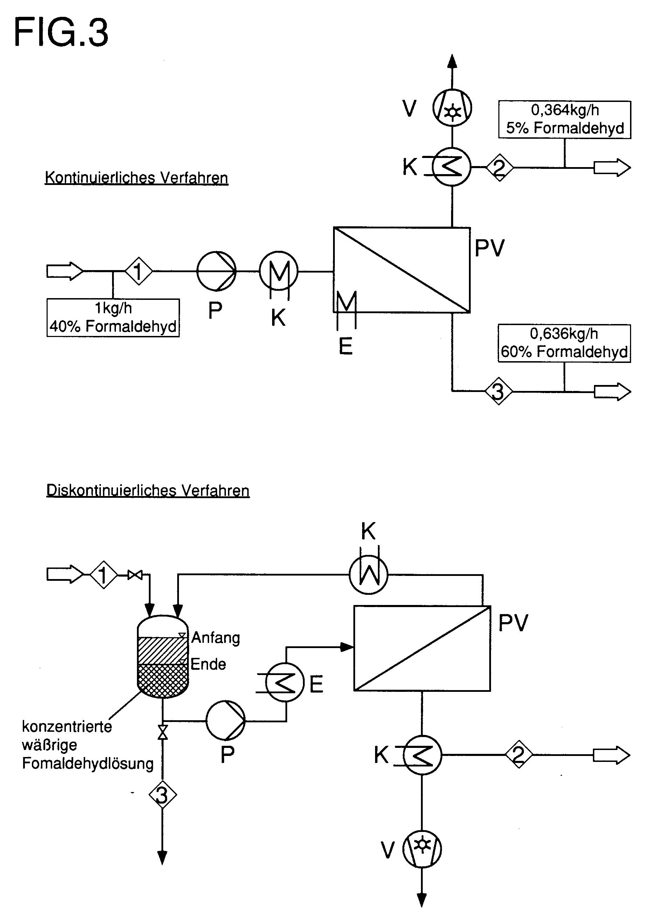

Figur 3 zeigt die Gewinnung konzentrierter wäßriger Formaldehydlösungen durch Pervaporation.

Im Unterschied zu den Ausführungsvarianten in Figur 1 und 2 wird die Formaldehyd-Rohlösung (Strom 1) in einem Verfahrensschritt durch Pervaporation in eine konzentrierte wäßrige Formaldehydlösung (Strom 3) und eine verdünnte wäßrige Lösung (Strom 2) überführt. Das Verfahren kann technisch sowohl in kontinuierlicher als auch in diskontinuierlicher Fahrweise betrieben werden. Die verfahrenstechnischen Vorteile dieser Variante sind, daß
- die Anzahl der Verfahrensschritte geringer und die apparative Ausführung einfacher ist als bei der Zweidruckdestillation.
- Die Wasserabtrennung energetisch günstiger ist als bei der Zweidruckdestillation und den zuvor beschriebenen Varianten.

### Beispiel 3

Mengen und Zusammensetzungen der einzelnen Ströme für die kontinuierliche Fahrweise sind in Figur 3 angegeben.

Die Pervaporation wird bei einer Temperatur von 80°C betrieben. Auf der Permeatseite wird ein Druck von 20 mbar gehalten. Verwendet wird das gleiche Membranmaterial wie im ersten Ausführungsbeispiel.

## Patentansprüche

1. Verfahren zur Gewinnung konzentrierter wäßriger Formaldehydlösungen durch Pervaporation, dadurch gekennzeichnet, daß ein wäßriges Formaldehydgemisch über eine hydrophile, porenfreie Membran geführt wird und selektiv Wasser über die Membran entfernt wird.

2. Verfahren nach Anspruch 1, dadurch gekennzeichnet, daß
- in einem ersten Verfahrensschritt durch Destillation die Rohlösung in einen konzentrierten Sumpf und ein wasserreiches Destillat aufgetrennt wird, und
- in einem zweiten Verfahrensschritt aus dem Destillat eine konzentrierte wäßrige Formaldehydlösung durch Pervaporation zurückgewonnen wird.

3. Verfahren nach Anspruch 1 und 2, dadurch gekennzeichnet, daß der wasserreiche Strom aus der Destillation dampfförmig über die Membran geführt wird und ein konzentrierter wäßriger Formaldehyddampf zurückgewonnen wird.

4. Verfahren nach Anspruch 1, dadurch gekennzeichnet, daß die Formaldehyd-Rohlösung in einem Verfahrensschritt durch Pervaporation in eine konzentrierte wäßrige Formaldehydlösung und eine verdünnte wäßrige Lösung überführt wird.
